# EUROPEAN PATENT SPECIFICATION

(11) **EP 1 853 197 B1**
(45) Date of publication and mention of the grant of the patent: **17.08.2011**
(21) Application number: 05801111.5
(22) Date of filing: 15.09.2005
(51) Int. Cl.: A61F 2/01

(54) **SHAPE MEMORY THIN FILM EMBOLIC PROTECTION DEVICE**
FORMGEDÄCHTNIS-DÜNNFILM-EMBOLIESCHUTZVORRICHTUNG
DISPOSITIF DE PROTECTION EMBOLIQUE A FILM MINCE A MEMOIRE DE FORME

(30) Priority: 17.09.2004 US 610899 P
(43) Date of publication of application: 14.11.2007
(73) Proprietor: Nitinol Development Corporation, Fremont, California 94539 (US)
(72) Inventor: DINH, Minh Q., Union City, California 94587 (US); RUSSELL, Scott M., San Jose, California 95125 (US)
(74) Representative: Belcher, Simon James
(86) International application number: PCT/US2005/032931
(87) International publication number: WO 2006/033958

(56) References cited:
- WO-A-03/015840
- US-A1- 2003 171 771
- US-A1- 2003 187 495

## Description

### Background of the Invention

### I. Field of the Invention

The present invention relates to the treatment of vascular disease by either percutaneous angioplasty and stenting or surgery. More particularly, the present invention relates to a system that reduces macro- and microembolization during the treatment of vascular disease. Even more particularly, the present invention is directed to a collapsible filter device wherein the filter element comprises a shape memory thin film.

### II. Discussion of the Related Art

A variety of surgical and non-surgical angioplasty procedures have been developed for removing obstructions from blood vessels. Balloon angioplasty utilizes a balloon-tipped catheter which may be inserted within a stenosed region of the blood vessel. By inflation of the balloon, the stenosed region is dilated. Stenting involves the permanent implantation of a metallic scaffold in the area of the obstruction, following balloon dilatation. The stent is often delivered on an angioplasty balloon, and is deployed when the balloon is inflated. Another alternative is the local delivery of medication via an infusion catheter. Other techniques, such as atherectomy, have also been proposed. In atherectomy, a rotating blade is used to shave plaque from an arterial wall. Finally, other techniques such as tissue ablation are sometimes performed to address electrical anomalies in heart rhythm. Surgery involves either removing the plaque from the artery or attaching a graft to the artery so as to bypass the obstructing plaque.

One problem common to all of these techniques is the accidental release of portions of the plaque or thrombus, resulting in emboli, which can lodge elsewhere in the vascular system. Such emboli may be dangerous to the patient, and may cause severe impairment of the distal circulatory bed. Depending upon the vessel being treated, this may result in a stroke or myocardial infarction or limb ischemia. Vascular filters or embolism traps for implantation into the vena cava of a patient are well known, being illustrated by, for example, U. S. Patents Nos. 4,727,873 and 4,688,553. Additionally, there is a substantial amount of medical literature describing various designs of vascular filters and reporting the results of the clinical and experimented use thereof. See, for example, the article by Eichelter & Schenk entitled "Prophylaxis of Pulmonary Embolism," Archives of Surgery, Vol. 97, August 1968, pp. 348 et seq. See, also, the article by Greenfield, et al., entitled "A New Intracaval Filter Permitting Continued Flow and Resolution of Emboli", Surgery, Vol. 73, No. 4, pp. 599-606 (1973).

Vascular filters are used, often during a postoperative period, when there is a perceived risk of a patient encountering a pulmonary embolus resulting from clots generated at the surgical site. Typically, the filter is mounted in the vena cava to catch large emboli passing from the surgical site to the lungs.

The vascular filters of the prior art are usually permanently implanted in the venous system of the patient, so that even after the need for the filter has abated, the filter remains in place for the lifetime of the patient, absent surgical removal. U.S. Patent No. 3,952,747 describes a stainless steel filtering device, which is permanently implanted transvenously within the inferior vena cava. The filtering device is intended to treat recurrent pulmonary embolism. U.S. Patent No. 4,873,978 describes a catheter device comprising a catheter body having a strainer mounted at its distal end. The strainer is shiftable between an opened configuration where it extends substantially across the blood vessel to entrap passing emboli, and a closed configuration where it retains the captured emboli during removal of the catheter.

A mechanism actuable at the proximate end of the catheter body allows selective opening and closing of the strainer. Typically, the strainer is a collapsible cone having an apex attached to a wire running from the distal end to the proximate end of the catheter body.

Permanent implantation may be deemed medially undesirable, but it has been done because vascular filters are implanted in patients primarily in response to potentially life threatening situations. Accordingly, the potential disadvantages of permanent implantations of a vascular filter are often accepted.

Notwithstanding the usefulness of the above-described methods, a need still exists for an apparatus and method for preventing embolization associated with conventional surgery and interventional procedures. In particular, it would be desirable to provide a device, which could be located within the vascular system, to collect and retrieve portions of plaque and thrombus which have dislodged during the surgery or angioplasty procedure.

WO-03/015840 relates to an embolic protection device. In one embodiment articulating arm members are formed by cutting slits into a catheter body member. A graft member is placed concentrically around a distal portion of the catheter body member, such that it is over the arm member and slits.

### SUMMARY OF THE INVENTION

The shape memory thin film embolic protection device with frame of the present invention overcomes the disadvantages associated with currently utilized devices.

In accordance with one aspect, the present invention is directed to a removable percutaneously delivered filter system as defined in appended claim 1. The percutaneously delivered filter system comprises a delivery system, including a sheath and a filter section operatively associated with the delivery system. The filter system also comprises a frame cooperatively associated with the filter section for adding radial strength. The filter section having a proximal end and a distal end. The proximal end having at least one opening allowing fluid to flow therethrough and the distal end having a multiplicity of pores for allowing fluid to flow therethrough while capturing particles of a predetermined size. The filter section being formed from a shape memory thin film material.

The present invention provides a vascular filter system useful in the surgical or interventional treatment of vascular disease. Macro- and microembolization may occur during percutaneous procedures such as angioplasty, which increases the risk of a minor or major stroke. The system of the present invention for reducing macro- and micro-embolization is very useful in helping to prevent the risk of stroke. However, this system would also be useful in any percutaneous angioplasty, stenting, thrombolysis or tissue ablation procedure, or surgical procedure where embolization is a risk. The vascular filter system of the present invention may decrease embolism while allowing brain, or other distal tissue, perfusion. The filters may be incorporated into a guidewire, which is used for the entire procedure from crossing a lesion to deploying a stent. Alternate delivery devices may also be utilized.

The shape memory thin film embolic protection device offers a number of advantages. The device is shaped like a non-compliant balloon that will ideally ensure one hundred percent wall opposition. The thin film with slotted pattern allows low profile configuration for delivery, especially if delivery is done without the frame in place. The thin film with slotted pattern allows more flexibility in the delivery sheath, especially if delivery is done without the frame in place. The outlet opening could be designed to smaller size to allow smaller capture profile. An increase of longitudinal length of the filter allows increased filter volume. Increased radiopacity is achieved by having larger cover surface areas of the filter.

### BRIEF DESCRIPTION OF THE DRAWINGS

The present invention will be apparent upon consideration of the following detailed description, taken in conjunction with the accompanying drawings, in which the reference characters refer to like parts throughout, and in which:
Figure 1 is a diagrammatic representation of a shape memory thin film embolic protection system having a frame structure within a vessel in accordance with the present invention.
Figure 2 is a diagrammatic representation of a shape memory filter frame in accordance with the present invention.
Figure 3 is a diagrammatic representation of a shape memory thin film embolic protection system having a frame structure in accordance with the present invention.

### DETAILED DESCRIPTION OF THE PREFERRED EMBODIMENTS

The present invention relates to a vascular filter system for use in percutaneous angioplasty and stenting, as well as, other vascular procedures as described herein, and provides for the prevention of distal embolism during vascular procedures. Further, the filter system of the present invention allows for distal perfusion while preventing embolization.

In accordance with one exemplary embodiment, the present invention is directed to a minimally invasive collapsible filter device for use in the field of medical procedures on vessels of the circulatory system. However, other uses are possible. The filter element is preferably made of metallic thin film via physical vapor deposition, or any other suitable process that shapes like an expanded balloon of a balloon catheter, and a Nitinol structural frame that fits inside the metallic thin film to help increase the radial resistance force. In this device, the structural frame is not attached to the thin film covering. Instead, the geometry of the covering is designed to fit over the structural frame "like a glove." Ideally, this improves manufacturing efficiencies. The device comprises multiple inlet openings at the proximal location to allow blood flow and outlet openings for example, a series of pores distally to filter blood clots and embolic material. The device is introduced into a vascular system in a collapsible configuration and delivered to its intended location through a guide catheter. The device is deployed and the filter expands across a blood vessel such that blood passing through the blood vessel is delivered through the filter element. A proximal inlet portion of the filter body has multiple inlet openings to allow blood and embolic material to enter the filter body, and a distal outlet portion of the filter body has a plurality of small outlet openings or pores to allow through-passage of blood, but to retain embolic material within the filter body.

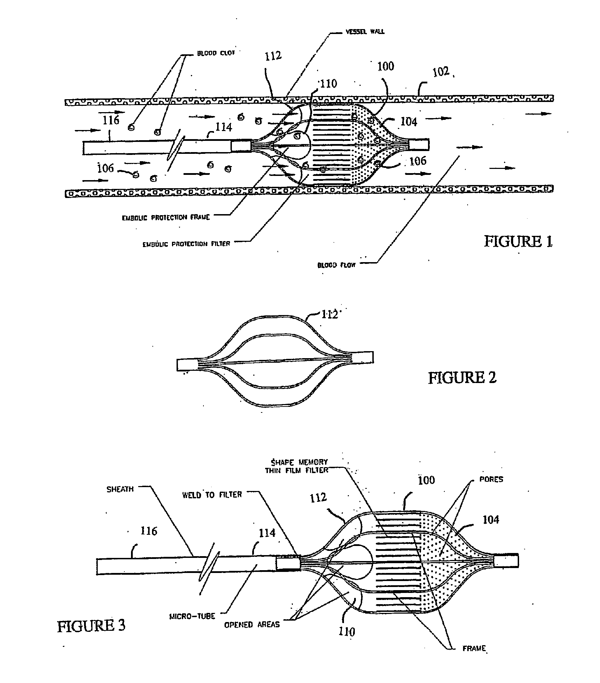

Figure 1 illustrates an exemplary shape memory thin film embolic protection device having a filter and frame 112 positioned within a vessel 102. The frame 112 fits fully within the filter. The shape memory thin film embolic protection system 100 comprises a filter having a distal end to capture embolic material or blood clots 106 flowing in the blood in the direction of arrows 108. Pores 104 in the distal end of the thin film allow blood to pass easily therethrough while capturing blood clots, particulates or other embolic material. The shape memory thin film embolic protection filter device 100 also comprises inlet openings 110 at its proximal end to allow blood to flow into the filter. The size of the inlet openings 110 may comprise any suitable configuration depending on the application. The shape memory thin film embolic protection device 100 also comprises a frame 112 to aid in increasing the radial resistance force. The shape memory thin film embolic protection device 100 may be connected to the delivery system via any number of means. In the illustrated exemplary embodiment, the thin film filter section and frame are fastened to a microtube 114 that is operatively associated with a catheter sheath 116. The fastening may be accomplished by any suitable means, including welding. Figure 2 illustrates the filter and frame 112 deployed within a vessel 102, whereas Figure 3 illustrates the entire device but not deployed within a vessel.

As stated above, the frame 112 is independent of the thin film comprising the filter, i.e., not be connected to the thin film, where the thin film filter slides over the frame 112.

The thin film material, as stated above, may be fabricated from Nitinol, . The material and design are subject to modification to ensure safety and efficacy. The material is designed from a shape memory material. The material may comprise a superelastic or Martensitic shape memory material and, in the preferred embodiment, the material comprises a nickel titanium alloy with about 50 to 60 weight percent nickel. The pores of the fabric are designed to capture particular matter in the size ranging from about 50µm to about 200µm.

In an alternate exemplary embodiment, the filter frame is at the end of an independent frame guidewire and the thin-film filtering device is at the end of a tubular filter guidewire with an inner diameter larger than the diameter of the frame guidewire. The frame and the filter and their associated guidewires are nested together for initial deployment. When a delivery sheath is retracted, the frame and the filter deploy simultaneously, with the frame inside the filter. Once deployment has been achieved, the frame may be collapsed and retracted through the center lumen of the hollow filter guidewire, leaving only the filter at the end of the filter guidewire remaining. The shape memory characteristics of the thin film filter ensure that it will retain its deployed shape even in the absence of the frame. Continuing acceptable wall opposition is maintained with the assistance of the blood flow through the filter. Once embolic protection is no longer needed, the filter guidewire may be withdrawn into a sheath, thereby collapsing the filter and containing the captured embolic material.

Another alternate exemplary embodiment allows the initial introduction of the filter without a frame. Subsequent to deployment of a frameless filter, a frame may be later deployed through the lumen in the filter guidewire to enhance wall opposition or to ensure complete deployment. The frame and its attached frame guidewire may be left in place during the procedure or removed as required. The advantage of this approach is that the distal protection device tends to have its lowest possible profile and maximum flexibility during initial crossing of the lesion, but has adequate support during procedural use.

The shape memory thin film embolic protection device offers a number of advantages. The device is shaped like a non-compliant balloon that ideally will ensure one hundred percent wall opposition. The thin film with slotted pattern allows low profile configuration for delivery, especially if delivery is done without the frame in place. The thin film with slotted pattern allows more flexibility in the delivery sheath, especially if delivery is done without the frame in place. The outlet opening could be designed to smaller size to allow smaller capture profile. An increase of longitudinal length allows increased basket volume. Increased radiopacity of the filter device is achieved by having larger cover surface areas comprising the filter and/or the frame.

Although shown and described is what is believed to be the most practical and preferred embodiments, it is apparent that departures from specific designs and methods described and shown will suggest themselves to those skilled in the art and may be used without departing from the scope of the invention. The present invention is not restricted to the particular constructions described and illustrated, but should be constructed to cohere with all modifications that may fall within the scope of the appended claims.

## Claims

1. A removable percutaneously delivered filter system comprising:
a delivery system, including a sheath (116);
a filter section operatively associated with the delivery system having a proximal end and a distal end, the proximal end having at least one opening (110) allowing fluid to flow therethrough and the distal end having a multiplicity of pores (104) for allowing fluid to flow therethrough and capturing particles of a predetermined size, the filter section being formed from a shape memory thin film material; and
a frame (112) cooperatively associated with the filter section for adding radial strength; **characterised in that** the frame (112) fits fully within the filter section and the frame (112) and the shape memory thin film material are not connected.

2. The vascular filter system of claim 1, wherein the filter and frame (112) are attached to the catheter delivery system such that retracting the sheath (116) deploys the filter and frame (112).

3. The vascular filter system of claim 1, wherein the catheter delivery system further comprises a frame guidewire having the frame (112) at an end thereof, and a tubular filter guidewire with the filter at an end thereof, the frame guidewire nestable within the tubular filter guidewire as deployment of the filter (112) and frame occurs.

4. The vascular filter system of claim 3, wherein the frame (112) is withdrawable through the tubular filter guidewire after deployment of the frame and filter.

5. The vascular filter system of claim 4, wherein the filter is withdrawable through the sheath (116) after withdrawal of the frame (112) and capture of embolic material.

6. The vascular filter system of any one of the preceding claims, wherein the filter section is shaped like an expanded balloon of a balloon catheter on deployment.

7. The vascular filter system of claim 6 further comprising a microtube (114) and wherein the filter section further comprises a slotted pattern between the at least one opening (110) and the multiplicity of pores (104), and the filter section and the frame (112) are fastened to the microtube (114).

## Patentansprüche

1. Entfernbares, perkutan zugeführtes Filtersystem, welches Folgendes umfasst:
ein Zuführungssystem, welches eine Hülle (116) aufweist;
ein wirksam mit dem Zuführungssystern verknüpfter Filterabschnitt, welcher ein proximales Ende und ein distales Ende aufweist, wobei das proximale Ende zumindest eine Öffnung (110) aufweist, wobei es einem Fluid ermöglicht ist, dort hindurch zu fließen, und wobei das distale Ende eine Vielzahl von Poren (104) aufweist, um es einem Fluid zu ermöglichen, dort hindurch zu fließen, und um Partikel einer vorbestimmten Größe aufzufangen, wobei der Filterabschnitt aus einem Dünnschichtmaterial mit Formgedächtnis gebildet ist; und
einen Rahmen (112), welcher zusammenwirkend mit dem Filterabschnitt verknüpft ist, um eine radiale Festigkeit hinzuzufügen; **dadurch gekennzeichnet, dass** der Rahmen (112) vollständig in den Filterabschnitt passt, und dass der Rahmen (112) und das Dünnschichtmaterial mit Formgedächtnis nicht verbunden sind.

2. Vaskularfiltersystem nach Anspruch 1, wobei das Filter und der Rahmen (112) an dem Katheterzuführungssystem angebracht sind, so dass ein Zurückziehen der Hülle (116) das Filter und den Rahmen (112) entfaltet.

3. Vaskularfiltersystem nach Anspruch 1, wobei das Katheterzuführungssystem weiter einen Rahmenführungsdraht mit dem Rahmen (112) an einem Ende davon und einen Röhrenfilterführungsdraht mit dem Filter an einem Ende davon umfasst, wobei der Rahmenführungsdraht in den Röhrenfilterführungsdraht steckbar ist, wenn eine Entfaltung des Filters (112) und des Rahmens geschieht.

4. Vaskularfiltersystem nach Anspruch 3, wobei der Rahmen (112) nach der Entfaltung des Rahmens und des Filters durch den Röhrenfilterführungsdraht zurückziehbar ist.

5. Vaskularfiltersystem nach Anspruch 4, wobei das Filter nach dem Zurückziehen des Rahmens (112) und dem Auffangen von embolischem Material durch die Hülle (116) zurückziehbar ist.

6. Vaskularfiltersystem nach einem der vorhergehenden Ansprüche, wobei der Filterabschnitt nach der Entfaltung wie ein ausgefalteter Ballon eines Ballonkatheters geformt ist.

7. Vaskularfiltersystem nach Anspruch 6, welches weiter einen Mikrotubus (114) umfasst, und wobei der Filterabschnitt weiter ein Schlitzmuster zwischen der zumindest einen Öffnung (110) und der Vielzahl von Poren (104) umfasst, und wobei der Filterabschnitt und der Rahmen (112) an dem Mikrotubus (114) befestigt sind.

## Revendications

1. Système de filtre amovible posé par voie percutanée comprenant :
un système de pose, comprenant une gaine (116) ;
une section de filtre associée fonctionnellement au système de pose, ayant une extrémité proximale et une extrémité distale, l'extrémité proximale ayant au moins une ouverture (110) permettant à un fluide de s'écouler à travers celle-ci, et l'extrémité distale ayant une multiplicité de pores (104) pour permettre à un fluide de s'écouler à travers ceux-ci et capturer des particules d'une taille prédéterminée, la section de filtre étant formée dans un matériau de film mince à mémoire de forme ; et
un cadre (112) associé coopérativement à la section de filtre pour ajouter une résistance radiale ; **caractérisé en ce que** le cadre (112) est logé entièrement à l'intérieur de la section de filtre et le cadre (112) et le matériau de film mince à mémoire de forme ne sont par liés.

2. Système de filtre vasculaire selon la revendication 1, dans lequel le filtre et le cadre (112) sont reliés au système de pose de cathéter de telle sorte que la rétractation de la gaine (116) déploie le filtre et le cadre (112).

3. Système de filtre vasculaire selon la revendication 1, dans lequel le système de pose de cathéter comprend en outre un fil-guide de cadre ayant le cadre (112) à une extrémité de celui-ci, et un fil-guide de filtre tubulaire avec le filtre à une extrémité de celui-ci, le fil-guide de cadre pouvant s'insérer à l'intérieur du fil-guide de filtre tubulaire lorsque le déploiement du filtre (112) et du cadre a lieu.

4. Système de filtre vasculaire selon la revendication 3, dans lequel le cadre (112) peut être retiré au moyen du fil-guide de filtre tubulaire après le déploiement du cadre et du filtre.

5. Système de filtre vasculaire selon la revendication 4, dans lequel le filtre peut être retiré au moyen de la gaine (116) après le retrait du cadre (112) et la capture de la matière embolique.

6. Système de filtre vasculaire selon l'une quelconque des revendications précédentes, dans lequel la section de filtre, une fois déployée, a la forme d'un ballonnet gonflé d'un cathéter à ballonnet.

7. Système de filtre vasculaire selon la revendication 6, comprenant en outre un microtube (114) et dans lequel la section de filtre comprend en outre un motif à fentes entre la au moins une ouverture (110) et la multiplicité de pores (104), et la section de filtre et le cadre (112) sont fixés sur le microtube (114).
